# EUROPEAN PATENT SPECIFICATION

(11) **EP 4 082 609 B1**
(45) Date of publication and mention of the grant of the patent: **18.12.2024**
(21) Application number: 21170669.2
(22) Date of filing: 27.04.2021
(51) Int. Cl.: A61N 5/10

(54) **OPTIMIZATION OF THERMORADIOTHERAPY TREATMENT**
OPTIMIERUNG DER THERMORADIOTHERAPIEBEHANDLUNG
OPTIMISATION D'UN TRAITEMENT PAR THERMORADIOTHÉRAPIE

(43) Date of publication of application: 02.11.2022
(73) Proprietor: RaySearch Laboratories AB, 104 30 Stockholm (SE)
(72) Inventor: Ödén, Jakob, 191 49 Sollentuna (SE); Eriksson, Kjell, 74651 Bålsta (SE); Fredriksson, Albin, 11869 Stockholm (SE); Traneus, Erik, 75239 Uppsala (SE)

(56) References cited:
- US-A1- 2017 216 623
- US-A1- 2018 304 100
- HANS CREZEE ET AL: "Thermoradiotherapy planning: Integration in routine clinical practice", INTERNATIONAL JOURNAL OF HYPERTHERMIA, vol. 32, no. 1, 2 January 2016 (2016-01-02), GB, pages 41 - 49, XP055326249, ISSN: 0265-6736, DOI: 10.3109/02656736.2015.1110757
- KOK H PETRA ET AL: "Hyperthermia Treatment Planning: Clinical Application and Ongoing Developments", IEEE JOURNAL OF ELECTROMAGNETICS, RF AND MICROWAVES IN MEDICINE AND BIOLOGY, IEEE, vol. 5, no. 3, 21 October 2020 (2020-10-21), pages 214 - 222, XP011873138, ISSN: 2469-7249, [retrieved on 20210819], DOI: 10.1109/JERM.2020.3032838

## Description

### Technical Field

The present invention relates to the planning of multimodality treatments combining radiation therapy and hyperthermia treatment. Such treatments are often referred to as thermoradiotherapy, or hyperthermia-enhanced radiotherapy.

### Background

Radiotherapy, or radiation therapy (RT), involves submitting a patient's body to radiation. Hyperthermia therapy (HT) refers to the treatment of malignant diseases by heating the tissue. Various heating techniques may be used, including, but not limited to, liquid agents, capacitive heating systems, exposure by electromagnetic radiation (radiofrequency, microwaves, or infrared), acoustic waves (ultrasound). The use of HT also enhances the effects of RT and some systemic types of treatment, such as chemotherapy. Raising the tumor temperatures to approximately 39-45°C, in combination with RT and/or systemic therapies has been found to increase the tumor control probability (TCP) compared to single modality treatments, and with practically no increase in late normal tissue complication probability (NTCP). This synergistic effect is due to various sensitization effects of HT, which include inhibition of DNA repair mechanisms and reoxygenation, as well as direct HT cell kill of radioresistant hypoxic tumor cells and triggering of multiple immune responses. The radiosensitizing effect can be quantified mathematically using known concepts like the equivalent radiation dose (EQD) including temperature-dependent parameters for the radiation, biological models including TCP and NTCP which could be dependent on the temperature-dependent EQD, the thermal enhancement ratio (TER), which is defined as the ratio of the radiation dose needed to produce a specific therapeutic effect without HT treatment, and the radiation dose needed to produce the same therapeutic effect combined with HT treatment.

Normally, different apparatuses are used for the HT treatment and the RT treatment, respectively, and for practical reasons they are therefore usually given sequentially to the patient, with approximately 0-4 h interval between the treatments. The delivery of different treatment modalities can also be simultaneous. RTHT treatments typically consists of approximately 5-35 RT fractions delivered daily, with a HT boost 1-2 times/week resulting in a total of approximately 1-10 HT fractions. Generally, the same RT treatment is delivered independently of whether or not a HT fraction is delivered on the same day, although strategies for adapting the RT plan on the days of HT delivery exist.

The optimization of such multimodality treatment involves the optimization of at least one RT plan and at least one HT plan. In this context, reference is made to document US2018/0304100 A1.

A general aim in all such treatments is to optimize the therapeutic effect of the treatment on the patient. For multimodality treatments, this involves exploiting the synergistic effects as much as possible. For a RTHT treatment, this includes exploiting effects related to co-localization of hot or cold temperature voxels, respectively, with RT doses, to account for the temperature-dependent radiosensitization in each voxel.

Accordingly, international patent application PCT/EP2016/074609 discloses a method of planning hyperthermia-enhanced radiation therapy in which a plan for either hyperthermia therapy or radiation therapy is first developed. After delivery of this plan to the patient, the result is evaluated and taken into account when optimizing the other plan. In other words, the hyperthermia treatment plan is optimized and delivered, and the radiation treatment plan is optimized taking the result of the delivery into account, or vice versa.

### Summary of the invention

The disclosure aims to provide an improved planning method for multimodality treatments involving both radiation therapy and hyperthermia therapy. The invention and its most advantageous embodiments are defined in the appended set of claims.

The disclosure relates to a computer-based method for generating a set of treatment plans including a radiation treatment plan and a hyperthermia treatment plan, for thermoradiotherapy treatment for a treatment volume of a patient, the method comprising the steps of:
a. obtaining an optimization problem including at least one objective function related to the multimodality treatment including hyperthermia and radiation, based on a model taking the combined predicted effect of heat and radiation dose into account,
b. generating at least one of the radiation treatment plan and the hyperthermia treatment plan by optimizing the optimization function value evaluated for the predicted combined effect over the set of treatment plans.

According to the invention, therefore, the synergistic effects of RTHT treatments can be enhanced, since treatments are optimized and planned together. The invention enables determining a more correct co-location of the temperature distribution, the radiation dose distribution, and optionally the effect of additional systemic therapies in the patient so that the synergistic effects of the treatments can be exploited more efficiently. In preferred embodiments, the radiation treatment plan is an external beam radiation treatment plan.

Issues related to the co-localization of hot or cold temperature voxels with corresponding RT doses can be mitigated, by increasing or reducing the dose in a voxel by accounting for the temperature-dependent sensitization of the radiation dose. In some cases, such issues may even be taken advantage of. For example, a radiation dose to a target may be reduced while maintaining a predetermined TCP if the temperature in the target is increased.

In an OAR a combination of high temperature and high radiation dose should be avoided, because the combination may result in an increased NTCP. This can be achieved by lowering the temperature and/or the radiation dose in the corresponding voxels in sequential optimization strategies, where the quantity of the modality optimized second is adjusted accounting for the combined predicted effect, or in a co-optimization strategy where both modalities are optimized simultaneously. In a target volume, the enhanced effect caused by a combination of high temperature and high radiation dose may be desired. In accordance with this, high temperatures and high radiation doses can be co-localized to the same voxel(s) within the target volume of the multimodality treatment to enhance the TCP using both sequential and co-optimization strategies.

Before performing step a), the method may include the step of defining the set of treatment plans including the hyperthermia plan and the radiotherapy plan and at least one other type of plan, such as surgery or a plan for a type of systemic treatment including chemotherapy, immunotherapy and hormone therapy. Alternatively, which plans to optimize are predetermined.

The set of treatment plans may for example comprise at least a first and a second radiotherapy plan, where the first radiotherapy plan is optimized for delivery on the same day as the at least one hyperthermia plan and the second radiotherapy plan is optimized for delivery on the days where another hyperthermia plan or no hyperthermia plan is delivered.

In addition to the RT and HT plans, the set of treatment plans comprises at least one additional therapy plan along with the at least one hyperthermia plan and at least one radiotherapy plan, said at least one additional therapy plan related to a systemic treatment such as chemotherapy.

Co-optimization of HT and RT is advantageous because these types of treatment are known to have a strong direct influence on each other and strong synergistic effects. The set of treatment plans may further include plans for one or more additional types of treatment. These additional types of treatment primarily include systemic therapies such as chemotherapy, hormone therapy and immunotherapy, but may also include surgery.

Three general embodiments have been devised, using the embodiment with one RT plan and one HT plan as an example: generating both the RT and the HT plan simultaneously, generating the RT plan and optimizing the HT plan in view of the predicted result of the RT plan, and optimizing the RT plan in view of the predicted result of the HT plan. In the first case, the method involves the step of generating the at least one of the radiation treatment plan and the hyperthermia treatment plan comprises co-optimization of the hyperthermia plan and the radiotherapy plan, the optimization problem including information on a combined predicted effect of temperature and dose for each voxel. In the second case, a pre-existing radiation treatment plan has been obtained and the step of generating the at least one of the radiation treatment plan and the hyperthermia treatment plan comprises optimizing the hyperthermia plan accounting for the predicted effect of at least one pre-existing radiotherapy plan, wherein the optimization problem includes the predicted effect of the radiation dose for each voxel. In the third case, a pre-existing hyperthermia plan has been obtained before the step of obtaining the optimization problem, and the step of generating the at least one of the radiation treatment plan and the hyperthermia treatment plan comprises optimizing the radiotherapy plan accounting for the predicted effect of at least one pre-existing hyperthermia plan, wherein the optimization problem includes temperature dependence information for the biological parameters for each voxel.

In addition to the one or more objective functions, the optimization problem may also comprise one or more constraints defining parameters that must be maintained during optimization. The optimization problem preferably comprises a biological or a physical objective. The optimization problem may also comprise at least one objective function for machine parameter optimization of one or both of the heat delivery system and the radiation delivery system. The optimization problem may also include at least one constraint related to machine limitations of at least one delivery machine that will be used to deliver hyperthermia treatment and/or radiation treatment as constraints or objectives. In some embodiments, the optimization problem includes a simplified machine model for either the heating system or the RT system for which the parameters are optimized, such as radiation fluence optimization and power optimization for the heating system.

The model used as a basis for the optimization problem may be a biological model. The optimization problem advantageously comprises a biological or a physical objective. The model and the objectives may relate to factors such as radiation dose, temperature, equivalent radiation dose (EQD), equivalent uniform distribution (EUD), biological effective dose (BED) and thermal enhancement ratio (TER) limits to targets and organs at risk (OAR) in the treatment volume, dose or temperature volume histograms (DVH and TVH) limits, probability limits for tumor control probability (TCP), and normal tissue complication probability (NTCP), complication free cure probability, secondary cancer and overall survival, linear energy transfer (LET) limits related to the radiation, the location where the particles stop and/or homogeneity and conformity indices.

Aspects of the invention also relate to a computer program product comprising computer-readable code which when run in a processor will cause the processor to perform the method according to the embodiments discussed above. The computer program product may be stored on non-transitory storage unit. Aspects of the invention also relate to a computer comprising a processor and a program memory, wherein the program memory holds such a computer program product for being executed in the processor.

### List of acronyms

The following acronyms are used in this document:
- BED - biological effective dose
- DVH - dose-volume histogram
- EQD - equivalent radiation dose
- EQD2 - equivalent radiation dose in 2-Gy fractions
- EUD - equivalent uniform dose
- HT - hyperthermia therapy
- LET - linear energy transfer
- LQ - linear-quadratic
- NTCP - normal tissue complication probability
- OAR - organ at risk
- RT - radiotherapy
- RTHT - thermoradiotherapy
- TCP - tumor control probability
- TER - thermal enhancement ratio. The ratio between the radiation dose required without HT and the radiation dose required for the same situation with HT
- TVH - temperature volume histogram.

### Brief description of drawings

The invention will be described in more detail in the following, by way of examples and with reference to the appended drawings.
Figures 1, 2, 3 are flow charts illustrating different embodiments of a method according to the invention.
Figure 4 is a schematic illustration of a computer system in which the method may be performed.

### Detailed description of embodiments

The methods according to embodiments of the invention relate to optimization of treatment plans. As is known in the art, such optimization is performed by applying an optimization problem that has been designed for the situation at hand, based on clinical goals and one or more biological models. The optimization problem is defined by objective functions, which define goals that the optimization should strive towards, for example minimizing dose to tissue surrounding a target, and constraints, which place absolute requirements, for example a minimum dose to the target. Constraints may also reflect machine limitations of at least one delivery machine that will be used to deliver hyperthermia treatment and/or radiation treatment. The skilled person is acquainted with such constraints, which include maximum speed of mechanical parts, maximum radiation delivery per time unit, and other limitations.

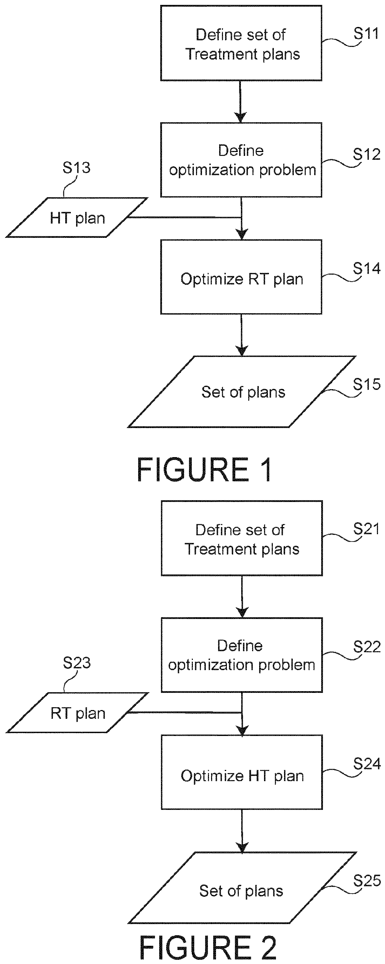

Figure 1 is a flow chart detailing a method according to a first embodiment of the disclosure, in which an RT plan is optimized considering the predicted effect of an HT plan that has been obtained in advance. In a first step S11, a thermoradiotherapy treatment is defined as a set of treatment plans corresponding to the treatment modalities involved. The set of plans in this embodiment includes a hyperthermia plan and a radiotherapy plan and may optionally also include plans of other modalities, such as surgery, immunotherapy, hormone therapy or chemotherapy. At this stage, the following information may be needed:
- the planned temperature distribution to result from HT,
- the time interval between the delivery of the two treatment modalities, if included in the model,
- the number of HT fractions and the delivery schedule, if not included in the optimization.

In a second step, S 12, an optimization problem is obtained for optimizing the set of plans together. To achieve this, in general, the optimization problem must include at least one objective function related to the combined treatment including hyperthermia and radiation, and optionally the other plan or plans included in the set. In this embodiment, the optimization problem comprises optimization functions where the temperature dependence of the biological parameters is included for each voxel of interest.

For example, the equivalent radiation dose (EQD) in 2-Gy fractions (EQD2) of the multimodality treatment could be optimized using the linear-quadratic (LQ) model for cell survival. The LQ-parameters α and β are known to vary with temperature as an increased temperature increase the radio sensitivity. By incorporating models for the temperature dependency of the LQ-parameter it is possible to optimize directly on the EQD2 for the combined predicted therapeutic effect of radiation dose and temperature. Objectives may be set for, for example, maximum/minimum EQD2, DVH limits as well as uniform EQD2. As a further development, EQD2 could be incorporated in one or more suitable models for optimization. Such models include equivalent uniform dose (EUD), tumor control probability (TCP) and normal tissue complication probability (NTCP) models. This could include using objective functions defined based on EQD2, including temperature dependency. It could also include using objective functions defined as models that are based on EQD2, including temperature dependency. In this manner the temperature dependent EQD2, EUD, TCP and/or NTCP will be optimized to fulfil the stated clinical goals.

A pre-existing temperature distribution of a hyperthermia plan is used as input for the optimization of a radiotherapy plan, and in step S 14, the RT plan is optimized while considering the radiosensitization caused by the temperature distribution resulting from the pre-existing HT plan S 13. The output S 15 of step S 14 is the set of plans including the pre-existing HT plan and the RT plan optimized using the optimization problem obtained in step S13. Modifications of the order of delivery, or of the number of fractions of the HT plan may be made to enhance the combined effect of RT and HT.

Figure 2 is a flowchart of a second embodiment of the method according to the invention, in which an HT plan is optimized while considering the combined effect with the radiation dose distribution resulting from a pre-existing RT plan. In a first step S21, a thermoradiotherapy treatment is defined as a set of treatment plans corresponding to the modalities of treatment involved. As for Figure 1, the set of plans includes a hyperthermia plan and a radiotherapy plan and may optionally also include other types of plans, such as surgery, immunotherapy, hormone therapy or chemotherapy, or a second radiotherapy plan related to a different type of radiotherapy.

In a second step, S22, an optimization problem is obtained for optimizing the set of plans together. To achieve this, the optimization problem must include at least one objective function related to the combined treatment including hyperthermia and radiation, and optionally the other plan or plans included in the set. In this embodiment, the objective function comprises optimization functions where the effect of the radiation dose resulting from the previously obtained radiotherapy plan is included for each voxel of interest. As in the method of Figure 1, optimization strategies using e.g., EQD2, TCP, and NTCP models could be used for the optimization of the hyperthermia plan.

A pre-existing dose distribution of a radiotherapy treatment plan is used as input for the optimization of a hyperthermia treatment plan, and in step S24, the HT plan is optimized while taking into account the dose distribution resulting from the pre-existing RT plan. The output S25 of step S24 is the set of treatment plans including the previously obtained RT plan and the HT plan optimized using the optimization problem obtained in step S22.

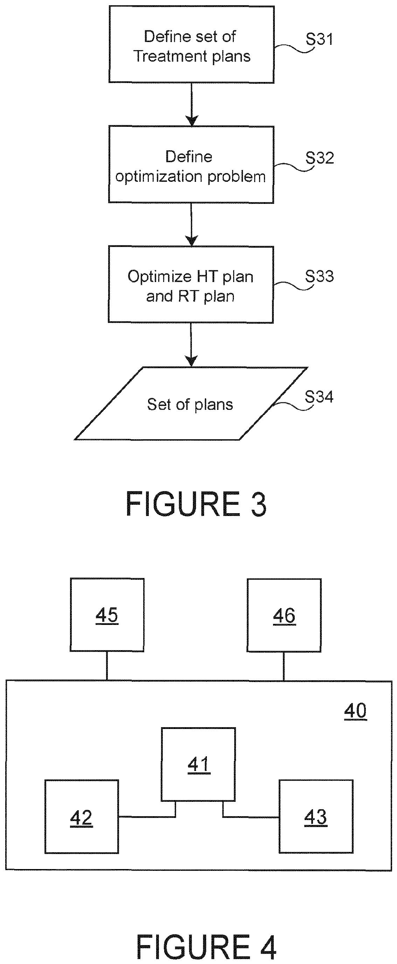

A third embodiment of the inventive method is shown in Figure 3. In a first step S31, a thermoradiotherapy treatment is defined as a set of treatment plans corresponding to the types of treatment involved. As in the previous embodiments, the set of plans includes one or more hyperthermia plans and one or more radiotherapy plans and may optionally also include other types of plans, such as surgery, immunotherapy, hormone therapy or chemotherapy.

In a second step, S32, an optimization problem is obtained for optimizing the set of plans together. To achieve this, the optimization problem must include at least one objective function related to the combined treatment including hyperthermia and radiation, and optionally the other plan or plans included in the set. In this embodiment, the objective function comprises optimization functions where the predicted combined effect of temperature and dose are included for each voxel of interest. As in the embodiments discussed above, optimization strategies using e.g., EQD2, EUD, TCP, and NTCP models could be used; however, in this embodiment the temperature and dose distributions are simultaneously co-optimized in the search for the optimal treatment in terms of the combined predicted therapeutic effect.

In step S33, the HT plan and the RT plan are optimized in one optimization operation. The output S34 of step S33 is the set of treatment plans including the HT plan and the RT plan optimized together using the optimization problem obtained in step S32.

The first step of each of the methods above, S11, S21, S31 may in many cases not be needed. It may be predefined which treatment plans are to be included, or the possible types of treatment may be restricted by the available equipment, for example. Co-optimization of HT and RT is advantageous because these types of treatment are known to have a strong direct influence on each other and strong synergistic effects.

In each of the methods discussed in connection with Figures 1, 2, 3 above, the step of obtaining the optimization problem may include retrieving an existing optimization problem or defining a new optimization problem, or adapting a predefined optimization problem to a specific situation by including or modifying objective functions and/or constraints.

Also, in each of the methods discussed above, two radiotherapy plans may be included in the set of treatment plans in addition to the hyperthermia treatment plan. Including two separate radiotherapy plans may be useful in the cases where two different types of radiation treatment should be combined. It is also possible to include a first radiotherapy plan including the fractions to be delivered in conjunction with hyperthermia treatments, and a second radiotherapy plan including the fractions to be delivered without the effect of hyperthermia treatment. In conjunction within this context means so near in time that the effects of the radiation and the hyperthermia overlap and enhance each other.

Figure 4 shows schematically a computer 40 in which the methods according to the invention may be performed. As is common in the art, the computer 40 comprises a processor 41, a program memory 42 and a data memory 43. As will be understood, the program and data memories 42, 43 may be implemented in any suitable way, in the form of one or more memories, internally or externally to the computer 40. The computer comprises, or is connected to, one or more user input/output devices, such as a screen, a keyboard, a mouse, audio input/output means, and any other suitable devices. The program memory 42 holds a computer program arranged to be run in the processor 41 to perform a method according to the invention, for example according to one of the embodiments discussed above. The data memory 43 or memories hold input data to be used in the method, such as patient data, information about the set of plans and, in the case of Figures 1 and 2, of the previously obtained plans used as input to the optimization. The data memory 43 may also hold the resulting set of plans that forms the output from the method.

The treatment plans may be planned for delivery by any suitable type of delivery equipment. As mentioned above, different apparatuses are normally used for delivery of HT and RT, respectively. The HT delivery apparatus may use any suitable technique, including, but not limited to, liquid agents, capacitive heating systems, exposure by electromagnetic radiation, acoustic waves (ultrasound), and magnetic HT where nanoparticles are injected in the tumor and subsequently heated by varying magnetic field over the tumor region. Electromagnetic radiation is often used and may include of electromagnetic radiation of any suitable wavelength, including radiofrequency, microwaves, or infrared.

The RT delivery apparatus is preferably an external beam radiation therapy device, for delivering any type of radiotherapy to a patient, including photon, electron or ion radiotherapy. Delivery apparatuses for both RT and HT are well known in the art and will not be discussed in more detail here. Also, the skilled person is familiar with apparatuses and equipment used for other types of treatments, such as surgery and systemic treatments, and their effects. Systemic treatments include chemotherapy, hormone therapy and immunotherapy, all of which are commonly used in the treatment of, for example, cancer patients.

## Claims

1. A computer-based method for generating a set of treatment plans including a radiation treatment plan and a hyperthermia treatment plan, for thermoradiotherapy treatment for a treatment volume of a patient, the method comprising the steps of:
a. obtaining (S 12; S22; S32) an optimization problem including at least one objective function related to the multimodality treatment including hyperthermia and radiation, based on a model taking the predicted combined effect of heat and radiation dose into account,
b. generating (S14; S24; S33) at least one of the radiation treatment plan and the hyperthermia treatment plan by optimizing the objective function value evaluated for the predicted combined effect over the set of treatment plans.

2. A method according to claim 1, wherein the radiation treatment plan is an external beam radiation treatment plan.

3. A method according to any one of the preceding claims, wherein the step of generating the at least one of the radiation treatment plan and the hyperthermia treatment plan comprises co-optimization (S33) of the hyperthermia plan and the radiotherapy plan, the optimization problem including information on a predicted combined effect of temperature and dose for each voxel.

4. A method according to any one of the claims 1-2, wherein a pre-existing hyperthermia plan has been obtained (S13) before the step of obtaining (S14) the optimization problem, and wherein the step of generating the at least one of the radiation treatment plan and the hyperthermia treatment plan comprises optimizing (S14) the radiotherapy plan accounting for the predicted effect of at least one pre-existing hyperthermia plan, wherein the optimization problem includes temperature dependence information for the biological parameters for each voxel.

5. A method according to any one of the claims 1-2, wherein a pre-existing radiation treatment plan has been obtained (S23) and wherein the step of generating the at least one of the radiation treatment plan and the hyperthermia treatment plan comprises optimizing (S24) the hyperthermia plan accounting for the predicted effect of at least one pre-existing radiotherapy plan, wherein the optimization problem includes the effect of the radiation dose for each voxel.

6. A method according to any one of the preceding claims, wherein the model includes one or more of the following: the equivalent radiation dose - EQD, equivalent uniform distribution - EUD, biological effective dose - BED, thermal enhancement ratio - TER, tumor control probability - TCP, normal tissue complication probability - NTCP, complication free cure probability, secondary cancer, and/or overall survival.

7. A method according to any one of the preceding claims, wherein the optimization problem comprises constraints which define parameters that are maintained during optimization.

8. A method according to any one of the preceding claims, wherein the optimization problem comprises a biological or a physical objective.

9. A method according to any one of the preceding claims, wherein the optimization problem is defined to optimize machine parameters of at least one of the heat delivery systems and the radiation delivery system as variables.

10. A method according to any one of the preceding claims, wherein the optimization problem includes at least one constraint related to machine limitations of at least one delivery machine that will be used to deliver hyperthermia treatment and/or radiation treatment as constraints or objectives.

11. A method according to any one of the preceding claims, wherein the optimization problem includes a simplified machine model for either the heating system or the RT system for which the parameters are optimized, such as radiation fluence optimization and power optimization for the heating system.

12. A method according to any one of the preceding claims, further comprising the step, performed before the steps of claim 1, of defining (S11; S21; S31) the set of treatment plans including the hyperthermia plan and the radiotherapy plan and at least one other type of plan, such as surgery or a plan for a type of systemic treatment including chemotherapy, immunotherapy and hormone therapy.

13. A method according to any one of the preceding claims, wherein the set of treatment plans comprises at least a first and a second radiotherapy plan, where the first radiotherapy plan is optimized for delivery on the same day as the at least one hyperthermia plan and the second radiotherapy plan is optimized for delivery on the days where no hyperthermia plan is delivered.

14. A method according to any one of the preceding claims, wherein the set of treatment plans comprises at least one additional therapy plan along with the at least one hyperthermia plan and at least one radiotherapy plan, said at least one additional therapy plan related to a systemic treatment such as chemotherapy.

15. A computer program product comprising computer-readable code means which when run in a processor will cause the processor to perform the method according to any one of the preceding claims.

16. A computer (40) comprising a processor (41) and a program memory (42),
wherein the program memory (42) holds a computer program product according to claim 14, for being executed in the processor (41).

## Patentansprüche

1. Computerbasiertes Verfahren zum Erzeugen eines Satzes von Behandlungsplänen, die einen Strahlenbehandlungsplan und einen Hyperthermiebehandlungsplan einschließen, für eine Thermoradiotherapiebehandlung für ein Behandlungsvolumen eines Patienten, das Verfahren umfassend die Schritte:
a. Erhalten (S12 ; S22 ; S32) eines Optimierungsproblems, das mindestens eine Zweckfunktion bezüglich der Multimodalitätsbehandlung einschließt, die Hyperthermie und Strahlung einschließt, basierend auf einem Modell, das die vorhergesagte kombinierte Wirkung von Wärme- und Strahlungsdosis berücksichtigt,
b. Erzeugen (S14 ; S24 ; S33) mindestens eines des Strahlenbehandlungsplans und des Hyperthermiebehandlungsplans durch ein Optimieren des Zweckfunktionswerts, der für die vorhergesagte kombinierte Wirkung über den Satz von Behandlungsplänen hinweg ausgewertet wird.

2. Verfahren nach Anspruch 1, wobei der Strahlenbehandlungsplan ein externer Strahlenbehandlungsplan ist.

3. Verfahren nach einem der vorstehenden Ansprüche, wobei der Schritt des Erzeugens mindestens eines des Strahlenbehandlungsplans und des Hyperthermiebehandlungsplans eine Co-Optimierung (S33) des Hyperthermieplans und des Strahlentherapieplans umfasst, wobei das Optimierungsproblem Informationen zu einer vorhergesagten kombinierten Wirkung von Temperatur und Dosis für jedes Voxel einschließt.

4. Verfahren nach einem der Ansprüche 1 bis 2, wobei ein bereits bestehender Hyperthermieplan erhalten (S13) wurde, vor dem Schritt des Erhaltens (S14) des Optimierungsproblems, und wobei der Schritt des Erzeugens mindestens eines des Strahlenbehandlungsplans und des Hyperthermiebehandlungsplans das Optimieren (S14) des Strahlentherapieplans unter Berücksichtigung der vorhergesagten Wirkung von mindestens einem bereits bestehenden Hyperthermieplan umfasst, wobei das Optimierungsproblem Temperaturabhängigkeitsinformationen für die biologischen Parameter für jedes Voxel einschließt.

5. Verfahren nach einem der Ansprüche 1 bis 2, wobei ein bereits bestehender Strahlenbehandlungsplan erhalten (S23) wurde und wobei der Schritt des Erzeugens des mindestens einen des Strahlenbehandlungsplans und des Hyperthermiebehandlungsplans das Optimieren (S24) des Hyperthermieplans unter Berücksichtigung der vorhergesagten Wirkung von mindestens einem bereits bestehenden Strahlentherapieplan umfasst, wobei das Optimierungsproblem die Wirkung der Strahlendosis für jedes Voxel einschließt.

6. Verfahren nach einem der vorstehenden Ansprüche, wobei das Modell eines oder mehrere der folgenden einschließt: die äquivalente Strahlungsdosis - EQD, äquivalente Gleichverteilung - EUD, biologische effektive Dosis - BED, thermisches Verstärkungsverhältnis - TER, Tumorkontrollwahrscheinlichkeit - TCP, Komplikationswahrscheinlichkeit für normales Gewebe - NTCP, Wahrscheinlichkeit einer komplikationsfreien Heilung, Sekundärkrebs und/oder Gesamtüberleben.

7. Verfahren nach einem der vorstehenden Ansprüche, wobei das Optimierungsproblem Einschränkungen umfasst, die Parameter definieren, die während der Optimierung beibehalten werden.

8. Verfahren nach einem der vorstehenden Ansprüche, wobei das Optimierungsproblem einen biologischen oder einen physischen Zweck umfasst.

9. Verfahren nach einem der vorstehenden Ansprüche, wobei das Optimierungsproblem definiert ist, um Maschinenparameter von mindestens einem des Wärmeverabreichungssystems und des Strahlungsverabreichungssystems als Variablen zu optimiert.

10. Verfahren nach einem der vorstehenden Ansprüche, wobei das Optimierungsproblem mindestens eine Einschränkung bezüglich Maschinenbeschränkungen von mindestens einer Verabreichungsmaschine einschließt, die verwendet wird, um eine Hyperthermiebehandlung und/oder Strahlenbehandlung als Einschränkungen oder Zwecke zu verabreichen.

11. Verfahren nach einem der vorstehenden Ansprüche, wobei das Optimierungsproblem ein vereinfachtes Maschinenmodell entweder für das Erwärmungssystem oder das RT-System einschließt, für das die Parameter optimiert sind, wie Strahlungsfluenzoptimierung und Leistungsoptimierung des Erwärmungssystems.

12. Verfahren nach einem der vorstehenden Ansprüche, ferner umfassend den Schritt, der vor den Schritten nach Anspruch 1 durchgeführt wird, des Definierens (S11 ; S21 ; S31) des Satzes von Behandlungsplänen, der den Hyperthermieplan und den Strahlentherapieplan und mindestens eine weitere Art von Plan einschließt, wie eine Operation oder einen Plan für eine Art von systemischer Behandlung, die Chemotherapie, Immuntherapie und Hormontherapie einschließt.

13. Verfahren nach einem der vorstehenden Ansprüche, wobei der Satz von Behandlungsplänen mindestens einen ersten und einen zweiten Strahlentherapieplan umfasst, wobei der erste Strahlentherapieplan für die Verabreichung an demselben Tag wie der mindestens eine Hyperthermieplan optimiert ist und der zweite Strahlentherapieplan für die Verabreichung an den Tagen optimiert ist, an denen kein Hyperthermieplan verabreicht wird.

14. Verfahren nach einem der vorstehenden Ansprüche, wobei der Satz von Behandlungsplänen neben dem mindestens einen Hyperthermieplan und dem mindestens einen Strahlentherapieplan mindestens einen zusätzlichen Therapieplan umfasst, wobei sich der mindestens eine zusätzliche Therapieplan auf eine systemische Behandlung bezieht, wie eine Chemotherapie.

15. Computerprogrammprodukt, umfassend computerlesbare Codemittel, die, wenn sie in einem Prozessor ablaufen, den Prozessor veranlassen, das Verfahren nach einem der vorstehenden Ansprüche durchzuführen.

16. Computer (40), umfassend einen Prozessor (41) und einen Programmspeicher (42), wobei der Programmspeicher (42) ein Computerprogrammprodukt nach Anspruch 14 für eine Ausführung in dem Prozessor (41) hält.

## Revendications

1. Procédé informatique permettant de générer un ensemble de plans de traitement comportant un plan de traitement par rayonnement et un plan de traitement par hyperthermie, pour un traitement de thermoradiothérapie pour un volume de traitement d'un patient, le procédé comprenant les étapes consistant à :
a. obtenir (S12 ; S22 ; S32) un problème d'optimisation comportant au moins une fonction objective se rapportant au traitement multimodal comportant une hyperthermie et un rayonnement, en fonction d'un modèle prenant en compte l'effet combiné prédit de la chaleur et de la dose de rayonnement,
b. générer (S14 ; S24 ; S33) au moins l'un parmi le plan de traitement par rayonnement et le plan de traitement par hyperthermie par optimisation de la valeur de fonction objective évaluée pour l'effet combiné prédit sur l'ensemble de plans de traitement.

2. Procédé selon la revendication 1, dans lequel le plan de traitement par rayonnement est un plan de traitement par rayonnement à faisceau externe.

3. Procédé selon l'une quelconque des revendications précédentes, dans lequel l'étape de génération de l'au moins un parmi le plan de traitement par rayonnement et le plan de traitement par hyperthermie comprend une cooptimisation (S33) du plan d'hyperthermie et du plan de radiothérapie, le problème d'optimisation comportant des informations relatives à un effet combiné prédit de la température et de la dose pour chaque voxel.

4. Procédé selon l'une quelconque des revendications 1 à 2, dans lequel un plan d'hyperthermie préexistant a été obtenu (S13) avant l'étape d'obtention (S14) du problème d'optimisation, et dans lequel l'étape de génération de l'au moins un parmi le plan de traitement par rayonnement et le plan de traitement par hyperthermie comprend l'optimisation (S14) du plan de radiothérapie en tenant compte de l'effet prédit d'au moins un plan d'hyperthermie préexistant, dans lequel le problème d'optimisation comporte des informations de dépendance à la température pour les paramètres biologiques pour chaque voxel.

5. Procédé selon l'une quelconque des revendications 1 à 2, dans lequel un plan de traitement par rayonnement préexistant a été obtenu (S23) et dans lequel l'étape de génération de l'au moins un parmi le plan de traitement par rayonnement et le plan de traitement par hyperthermie comprend l'optimisation (S24) du plan d'hyperthermie en tenant compte de l'effet prédit d'au moins un plan de radiothérapie préexistant, dans lequel le problème d'optimisation comporte l'effet de la dose de rayonnement pour chaque voxel.

6. Procédé selon l'une quelconque des revendications précédentes, dans lequel le modèle comporte un ou plusieurs de ce qui suit : dose de rayonnement équivalente - EQD, distribution uniforme équivalente - EUD, dose efficace biologique - BED, rapport de renforcement thermique - TER, probabilité de maîtrise de tumeur - TCP, probabilité de complication de tissu normal - NTCP, probabilité de guérison sans complication, cancer secondaire, et/ou survie globale.

7. Procédé selon l'une quelconque des revendications précédentes, dans lequel le problème d'optimisation comprend des contraintes qui définissent des paramètres qui sont maintenus pendant l'optimisation.

8. Procédé selon l'une quelconque des revendications précédentes, dans lequel le problème d'optimisation comprend un objectif biologique ou physique.

9. Procédé selon l'une quelconque des revendications précédentes, dans lequel le problème d'optimisation est défini pour optimiser des paramètres de machine d'au moins l'un parmi les systèmes d'administration de chaleur et le système d'administration de rayonnement en guise de variables.

10. Procédé selon l'une quelconque des revendications précédentes, dans lequel le problème d'optimisation comporte au moins une contrainte se rapportant à des limitations de machine d'au moins une machine d'administration qui sera utilisée pour administrer un traitement par hyperthermie et/ou un traitement par rayonnement en guise de contraintes ou d'objectifs.

11. Procédé selon l'une quelconque des revendications précédentes, dans lequel le problème d'optimisation comporte un modèle de machine simplifié soit pour le système de chauffage soit pour le système RT pour lequel les paramètres sont optimisés, tel qu'une optimisation de fluence de rayonnement et une optimisation de puissance pour le système de chauffage.

12. Procédé selon l'une quelconque des revendications précédentes, comprenant en outre l'étape, mise en oeuvre avant les étapes de la revendication 1, consistant à définir (S11 ; S21 ; S31) l'ensemble de plans de traitement comportant le plan d'hyperthermie et le plan de radiothérapie et au moins un autre type de plan, tel qu'une intervention chirurgicale ou un plan pour un type de traitement systémique comportant une chimiothérapie, une immunothérapie et une thérapie hormonale.

13. Procédé selon l'une quelconque des revendications précédentes, dans lequel l'ensemble de plans de traitement comprend au moins un premier et un second plan de radiothérapie, où le premier plan de radiothérapie est optimisé pour administration le même jour que l'au moins un plan d'hyperthermie et le second plan de radiothérapie est optimisé pour administration les jours où aucun plan d'hyperthermie n'est administré.

14. Procédé selon l'une quelconque des revendications précédentes, dans lequel l'ensemble de plans de traitement comprend au moins un plan de thérapie supplémentaire en même temps que l'au moins un plan d'hyperthermie et au moins un plan de radiothérapie, ledit au moins un plan de thérapie supplémentaire se rapportant à un traitement systémique tel qu'une chimiothérapie.

15. Produit programme d'ordinateur comprenant un moyen de code lisible par ordinateur qui lorsqu'il est exécuté dans un processeur amènera le processeur à mettre en oeuvre le procédé selon l'une quelconque des revendications précédentes.

16. Ordinateur (40) comprenant un processeur (41) et une mémoire de programme (42), dans lequel la mémoire de programme (42) contient un produit programme d'ordinateur selon la revendication 14, destiné à être exécuté dans le processeur (41).
